# EUROPÄISCHE PATENTANMELDUNG

(11) **EP 0 581 079 A1**
(43) Veröffentlichungstag der Anmeldung: **02.02.1994**
(21) Anmeldenummer: 93110936.7
(22) Anmeldetag: 08.07.1993
(51) Int. Cl.: C07D 211/90, A61K 31/44

(54) **Spezieller, 1,4-Dihydropyridin-3,5-dicarbonsäureester, Verfahren zu seiner Herstellung und seine pharmazeutische Verwendung**

(30) Priorität: 20.07.1992 DE 4223867
(71) Anmelder: BAYER AG, 51368 Leverkusen (DE)
(72) Erfinder: Behner, Otto, Dr., D-42113 Wuppertal (DE); Wollweber, Hartmund, Dr., D-42113 Wuppertal (DE); Rosen, Bruno, Dr., D-42489 Wülfrath (DE); Zaiss, Siegfried, Dr., D-42113 Wuppertal (DE); Goldmann, Siegfried, Dr., D-42327 Wuppertal (DE)

(57) **Zusammenfassung**

Die Erfindung betrifft den neuen 4-(4-Chlor-3-trifluormethylphenyl)-1-cyclopropyl-2,6-dimethyl-1,4-dihydropyridin-3,5-dicarbonsäuredimethylester der allgemeinen Formel (I) 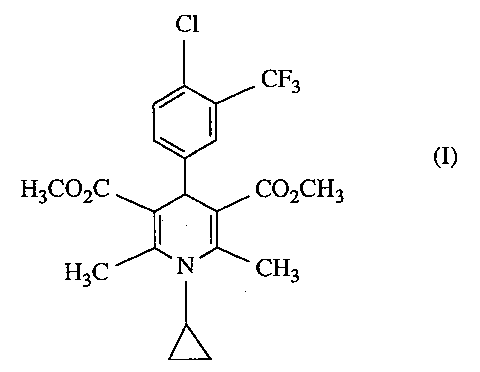
Verfahren zu seiner Herstellung und seine Verwendung als Arzneimittel bei ischämischen Erkrankungen, die mit Mikrozirkulationsstörungen verbunden sind. Diese Wirkung kann sowohl im peripheren als auch im cerebralen Gefäßsystem auftreten.

## Beschreibung

Die Erfindung betrifft den neuen 4-(4-Chlor-3-trifluormethylphenyl)-1-cyclopropyl-2,6-dimethyl-1,4-dihydropyridin-3,5-dicarbonsäuredimethylester, Verfahren zu seiner Herstellung und seine Verwendung als Arzneimittel bei ischämischen Erkrankungen, die mit Mikrozirkulationsstörungen verbunden sind. Diese Wirkung kann sowohl im peripheren als auch im cerebralen Gefäßsystem auftreten.

Es ist bereits bekannt, daß 1,4-Dihydropyridindicarbonsäureester eine calciumantagonistische oder calciumagonistische Wirkung besitzen, und somit als gefaß- und kreislaufbeeinflussende Mittel eingesetzt werden können (vgl. US 3 773 773).

In der EP 240 828 (US 4 975 440) werden auch blutdrucksenkende 1,4-Dihydropyridine mit hämorheologischen Eigenschaften beschrieben.

Die vorliegende Erfindung betrifft den neuen 4-(4-Chlor-3-trifluormethylphenyl)-1-cyclopropyl-2,6-dimethyl-1,4-dihydropyridin-3,5-dicarbonsäuredimethylester der Formel (I), 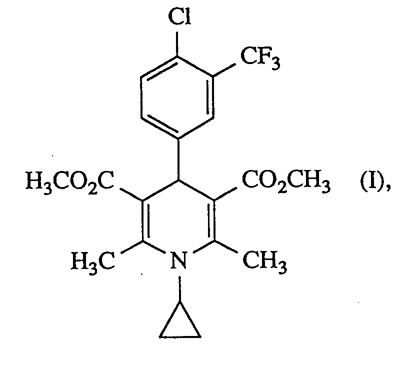
der überraschenderweise eine starke hämorheologische Wirkung besitzt und die Durchblutung, insbesondere die Mikrozirkulation verbessert und gleichzeitig blutdruckneutral ist. Er ist somit besonders geeignet zur Verwendung bei der Bekämpfung akuter und chronisch ischämischer Erkrankungen.

Die erfindungsgemäße Verbindung der Formel (I) kann nach üblichen Methoden hergestellt werden, z.B. indem man
[A] (4-Chlor-3-trifluormethylbenzyliden)acetessigsäuremethylester (II) 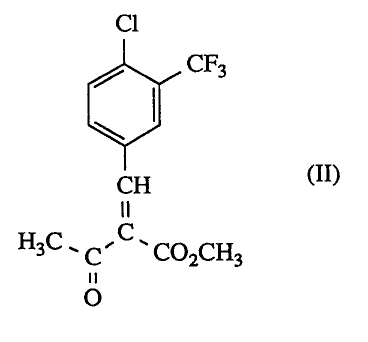 entweder direkt mit 3-Cyclopropylaminocrotonsäuremethylester, oder Acetessigsäuremethylester und Cyclopropylamin-Hydrochlorid, in inerten Lösemitteln, gegebenenfalls in Anwesenheit einer Base/Säure, umsetzt,
oder
[B] 4-Chlor-3-trifluormethylbenzaldehyd der Formel (III) 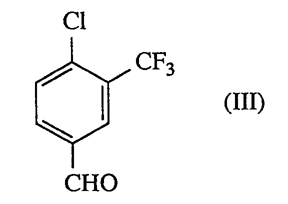 entweder mit Acetessigsäuremethylester und Cyclopropylamin-Hydrochlorid bzw. Cyclopropylamin und Pyridin-Hydrochlorid in Pyridin umsetzt,
oder
[C] zunächst unter Schutzgasatmosphäre Lewissäuren, vorzugsweise Titantetrachlorid, mit einer Base, vorzugsweise Piperidin, mit 3-Cyclopropylaminocrotonsäuremethylester in inerten Lösemitteln versetzt und anschließend 4-Chlor-3-trifluormethylbenzaldehyd der Formel (III) zufügt.

Das erfindungsgemaße Verfahren kann durch folgendes Formelschema beispielhaft erläutert werden: 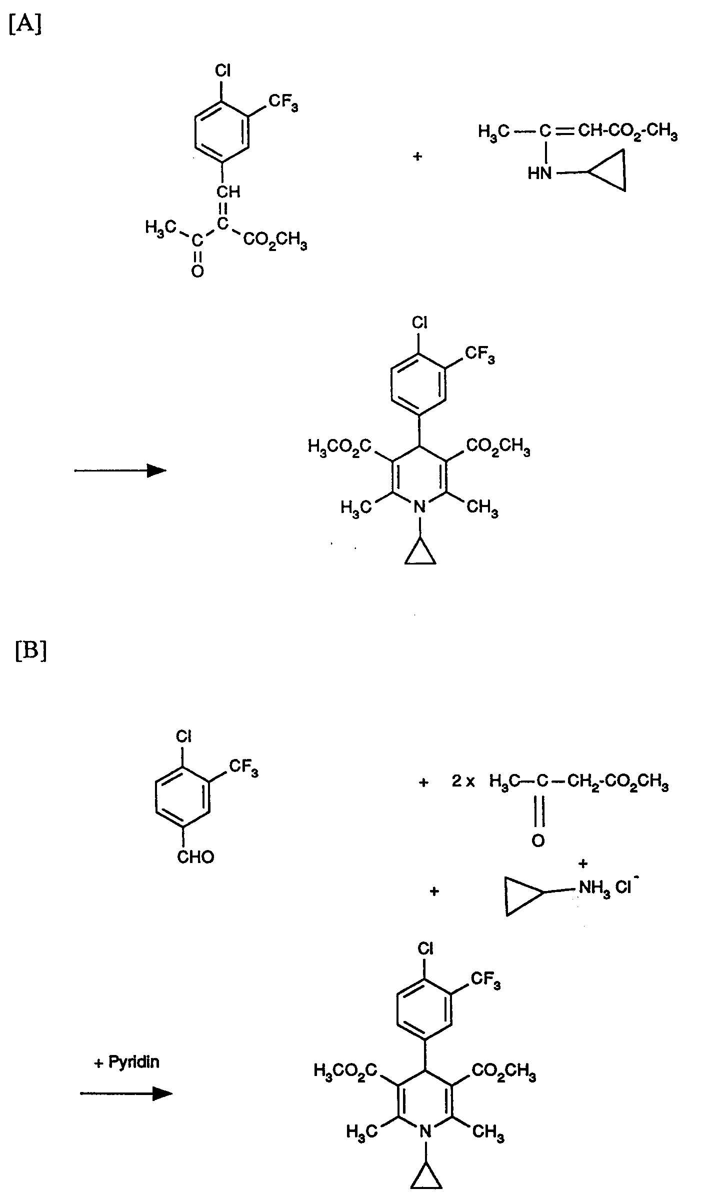 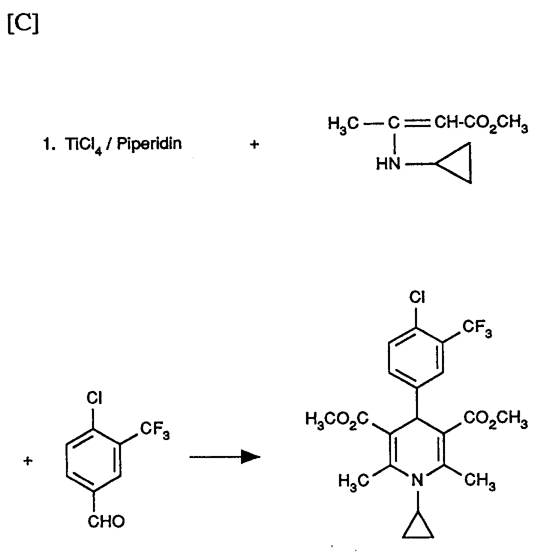
Als Lösemittel kommen Wasser oder organische Lösemittel in Frage, die sich unter den Reaktionsbedingungen nicht verändern. Hierzu gehören vorzugsweise Alkohole wie Methanol, Ethanol, Propanol, Isopropanol, Butanol, Ether wie Diethylether, Dioxan, Tetrahydrofuran, Glykolmonomethylether oder Glykoldimethylether, oder Amide wie Dimethylformamid, Dimethylacetamid oder Hexamethylphosphorsäuretriamid, oder Eisessig, Dimethylsulfoxid, Acetonitril oder Pyridin. Bevorzugt sind 1,2-Dimethoxyethan, Butanol und Pyridin.

Als Basen können in Abhängigkeit von den einzelnen Verfahrensschritten Hydride wie Natriumhydrid, Alkali- oder Erdalkalihydroxide, wie beispielsweise Natriumhydroxid oder Kaliumhydroxid, Alkoholate, wie Kalium-tert.butylat, oder Pyridin eingesetzt werden. Bevorzugt sind Natriumhydrid und Pyridin.

Als Säuren werden im allgemeinen Salzsäure oder Schwefelsäure eingesetzt.

Die Reaktionstemperaturen können in einem größeren Bereich variiert werden. Im allgemeinen arbeitet man zwischen +10°C und +150°C, vorzugsweise zwischen +20°C und +120°C, insbesondere bei der Siedetemperatur des jeweiligen Lösemittels.

Die Umsetzung kann bei Normaldruck, aber auch bei erhöhtem oder erniedrigtem Druck durchgeführt werden. Im allgemeinen arbeitet man bei Normaldruck.

Bei der Durchführung der erfindungsgemäßen Verfahrensvarianten A, B und C ist das Verhältnis der an der Reaktion beteiligten Stoffe beliebig. Im allgemeinen arbeitet man jedoch mit molaren Mengen der Reaktanden. Die Isolierung und Reinigung der erfindungsgemäßen Substanz erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkristallisiert. In einigen Fällen kann es erforderlich sein, die erfindungsgemäße Verbindung durch Chromatographie zu reinigen.

Die Verbindungen der Formeln (II) und (III) sind bekannt.

Die neue erfindungsgemäße Verbindung zeigt ein nicht vorhersehbares, wertvolles pharmakologisches Wirkspektrum. Während sie ein gefäß- und blutdruckneutrales Verhalten in einem Dosis-Bereich bis mindestens 10 mg/kg i.v. und 30 mg/kg p.o. zeigt, steigert sie gleichzeitig die Durchblutung, insbesondere die Mikrozirkulation durch Beeinflussung der Verformbarkeit der Erythrozyten sowie der Inhibition der Aktivierung und Adhäsion der Leukozyten.

Die Blutdruckneutralität wird an folgenden, für Dihydropyridine typischen Modellen, ermittelt: In SH-Ratten nach p.o. Gabe durch Messung an der Schwanzarterie (Riva Rocci Methode) und an narkotisierten Wistar-Ratten nach i.v. Gabe. Die Messung erfolgte über Katheter in A. carotis. Als blutdruckneutral wird diese Verbindung bezeichnet, da sie in beiden Testmodellen mit der angegebenen Dosis den Blutdruck nicht senkt. Der Abstand der therapeutischen Dosis und einer Blutdruckwirkung ist mindestens Faktor 100.

Die erfindungsgemäße Verbindung kann deshalb für die Herstellung von Arzneimitteln zur Behandlung von akuten und chronisch ischämischen Erkrankungen, wie Claudicatio intermittens, Myokardinfarkt, Gehirninfarkt sowie von Reperfusionsschäden und Schock eingesetzt werden.

Die folgenden in vitro und in vivo Tests zeigen die interessanten Wirkungen der speziell ausgewählten erfindungsgemäßen Verbindung.

### I) Erythrozytenfunktion

Die Verformbarkeit von Erythrozyten spielt für die Entstehung und den Verlauf akuter oder chronisch ischämischer Erkrankungen eine wesentliche Rolle. Sie bestimmen die Viskosität des Bluts und damit seine Verteilung in der Mikrozirkulation. Die angewendeten Tests erfassen verschiedene Determinanten:
Test a) erfaßt die antihämolytische Wirkung der Substanzen (ED₅₀, mol/l). Hierbei werden calciumbeladene Erythrozyten unter hohen Schubspannungen durch kleine Poren gepreßt, so daß Hämoglobin als Ausdruck ihrer Hämolyse freigesetzt und gemessen wird. Die Verminderung der Hämoglobinfreisetzung ist die Meßgröße.
Test b) erfaßt die Viskosität von Erythrozytensuspensionen in Glaskapillaren (25 µm Durchmesser) bei niedrigen, in Gefäßgebieten hinter einer Stenose auftretenden Schubspannungen. Durch Erhöhung des extrazellulären Calciums steigt die Viskosität an.

### a) Antihämolytische Wirkung an Erythrozyten

Normale Erythrozyten werden unter hohen Schubspannungen hämolytisch. Besonders ausgeprägt ist die Hämolyse von calciumbeladenen Zellen. Dieses Maß für mechanische Stabilität wird zur Substanzcharakterisierung herangezogen. Meßgröße ist die Konzentration freien Hämoglobins im Medium. Die MEK beträgt 5 x 10⁻⁷ mol/l.

### b) Viskosität in Glaskapillaren

Die biophysikalischen, für die Durchblutung relevanten Wechselwirkungen von Erythrozyten können in Glaskapillaren (Durchmesser 20-30 µm) untersucht werden. Die resultierende Viskosität hängt von dem Zustand der Zellen ab. Bei Calciumbeladung steigt die Viskosität an. Angegeben ist die prozentuale Verbesserung der Viskosität bezogen auf geschädigte, jedoch unbehandelte Kontrolle bei 0,7 Pa. Die Testdosis beträgt 10⁻⁸ g/ml.

**Tabelle I**

| Beispiel-Nr. | Effekt (%) |
|---|---|
| erfindungsgemäße Verbindung (I) | 72 |

### II) Leukozytenfunktion

Im Modell der Hamsterbackentasche kann die Mikrozirkulation direkt beobachtet werden. Meßgrößen sind die Leukozytenadhäsion sowie Gefäßdurchmesser und Erythrozytensenkungsgeschwindigkeit. Die Adhäsion wurde unter ischämischen und nicht ischämischen Versuchsbedingungen quantifiziert. Unter nicht ischämischen Bedingungen ist die Adhäsion im Bereich kleiner Venulen quantifiziert, unter ischämischen Bedingungen (10 min Durchblutungsstop) in kleinen Arteriolen. Die Ergebnisse der Kontrollversuche sind auf 100 % angeglichen. Als Testdosis werden jeweils 0,1 mg/kg i.v. gewählt, die Ergebnisse sind Abnahme in % der Kontrolle. Überraschenderweise zeigte sich, daß unter ischämischen Bedingungen die Substanz auch noch mit 0,1 mg/kg i.v. wirkte. Dies ist besonders günstig für die angestrebten Indikationen.

**Tabelle II**

| Beispiel-Nr. | nicht-ischämisch Kontrolle = 100% | ischämisch Kontrolle = 100% |
|---|---|---|
| erfindungsgemäße Verbindung (I) | 50% | 49% |

### III) Blutdruck

Der klinische Kenntnisstand zeigt, daß antiischämische Wirkungen von Dihydropyridinen häufig durch eine Vasodilatation überdeckt werden. Es war daher Ziel, blutdruckunwirksame (d.h. Abstand hämorheologische Wirkung und blutdrucksenkende Wirkung ≧ 10) DHP's zu finden. Die folgende Tabelle zeigt die Dosen, bei denen bei p.o.-Gabe (SH-Ratte) bzw. i.v.-Gabe (narkotisierte Wistaratte) eine Blutdrucksenkung eintritt.

**Tabelle III**

| Beispiel-Nr. | p.o.(mg/kg) | i.v(mg/kg) |
|---|---|---|
| erfindungsgemäße Verbindung (I) | >90 | >10 |

Die Tabelle zeigt, daß im Vergleich mit Modell II der Abstand der therapeutischen Wirkung und Blutdruckwirkung (i.v.) mindestens 100 beträgt.

Der neue Wirkstoff kann in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Herstellungsbeispiele

### Beispiel 1

### 4-(4-Chlor-3-trifluormethylphenyl)-1-cyclopropyl-2,6-dimethyl-1,4-dihydropyridin-3,5-dicarbonsäuredimethylester

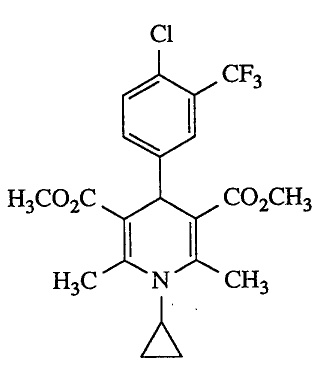

### Verfahren A

Eine Lösung von 23,3 g (0,15 mol) 3-Cyclopropylamino-crotonsäuremethylester, 46,0 g (0,15 mol) (4-Chlor-3-trifluormethylbenzyliden)-acetessigsäuremethylester und 17,7 g (0,15 mol) Pyridin-hydrochlorid in 100 ml Pyridin wird 7,5 h unter Rückfluß gerührt. Nach dem Abdampfen im Vakuum wird der Rückstand zwischen 120 ml Methylenchlorid und 150 ml Wasser verteilt. Die organische Phase gibt nach dem Eindampfen ein harziges Rohprodukt, das aus 100 ml Cyclohexan umkristallisiert wird.
Schmp.: 131-135°C
Ausbeute: 35,0g (52,6% d.Th.)

### Verfahren B

Eine Lösung von 4,17 g (0,02 mol) 4-Chlor-3-trifluormethylbenzaldehyd, 4,65 g (0,04 mol) Acetessigsäuremethylester, 1,96 g (0,021 mol) Cyclopropylamin-Hydrochlorid in 20 ml Pyridin wird 4 h unter Rückfluß gerührt. Nach dem Eindampfen wird der Rückstand zwischen 40 ml Methylenchlorid und 50 ml Wasser verteilt. Aus der organischen Phase erhält man durch Eindampfen ein öliges Rohprodukt, das mit Cyclohexan und n-Pentan zur Kristallisation gebracht wird.
Schmp.: 131-134°C
Ausbeute: 3,33 g (37,5% d.Th.)

## Patentansprüche

1. 4-(4-Chlo-3-trifluormethylphenyl)-1-cyclopropyl-2,6-dimethyl-1,4-dihydropyridin-3,5-dicarbonsäuredimethylester.

2. Verfahren zur Herstellung des 4-(4-Chlor-3-trifluormethylphenyl)-1-cyclopropyl-2,6-dimethyl-1,4-dihydropyridin-3,5-dicarbonsäuredimethylester, dadurch gekennzeichnet, daß man
[A] (4-Chlor-3-trifluormethylbenzyliden)acetessigsäuremethylester (II) 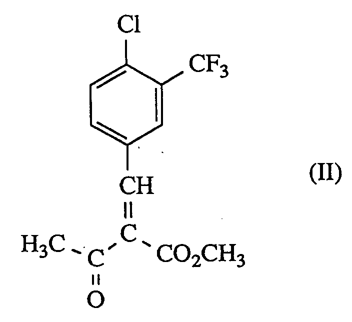 entweder direkt mit 3-Cyclopropylaminocrotonsäuremethylester, oder Acetessigsäuremethylester und Cyclopropylamin-Hydrochlorid,
in inerten Lösemitteln, gegebenenfalls in Anwesenheit einer Base/Säure, umsetzt,
oder
[B] 4-Chlor-3-trifluormethylbenzaldehyd der Formel (III) 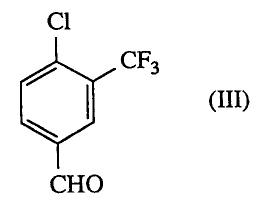 entweder mit Acetessigsäuremethylester und Cyclopropylamin-Hydrochlorid bzw. Cyclopropylamin und Pyridin-Hydrochlorid in Pyridin umsetzt,
oder
[C] zunächst unter Schutzgasatmosphäre Lewissäuren, vorzugsweise Titantetrachlorid, mit einer Base, vorzugsweise Piperidin, mit 3-Cyclopropylaminocrotonsäuremethylester in inerten Lösemitteln versetzt und anschließend 4-Chlor-3-trifluormethylbenzaldehyd der Formel (III) zufügt.

3. Arzneimittel, enthaltend die Verbindung gemäß Anspruch 1.

4. Verfahren zur Herstellung von Arzneimitteln, dadurch gekennzeichnet, daß man die Verbindung gemäß Anspruch 1 gegebenenfalls unter Zusatz üblicher Hilfs- und Trägerstoffe in eine geeignete Applikationsform überführt.

5. Verwendung der Verbindung gemäß Anspruch 1 zur Behandlung von Mikrozirkulationsstörungen.
